# EUROPEAN PATENT APPLICATION

(11) **EP 2 823 789 A1**
(43) Date of publication of application: **14.01.2015**
(21) Application number: 14171025.1
(22) Date of filing: 03.06.2014
(51) Int. Cl.: A61F 2/16

(54) **Small aperture (pinhole) intraocular implant to increase depth of focus**

(30) Priority: 19.06.2013 BR 102013015451
(71) Applicant: Trindade, Claudio Lovaglio Cancado, 30210-510 Belo Horizonte - MG (BR)
(72) Inventor: Trindade, Claudio Lovaglio Cancado, 30210-510 Belo Horizonte - MG (BR)
(74) Representative: Spachmann, Holger

(57) **Abstract**

**Small aperture (pinhole) intraocular implant to increase depth of focus** comprising a diaphragm (21, 21', 21 ") juxtaposed to the front surface of a lens implanted previously, having its anterior surface convex and its posterior surface concave. The diaphragm (21, 21', 21 ") is held in position by inserting engaging means (25, 25', 25 ") in the ciliary sulcus (13).

It is proposed that said diaphragm is opaque to a visible light spectrum and transparent to light in the infrared range and is equipped with passage means (23) of visible light in its central region, such as a through hole whose diameter is between 1 mm and 2.5 mm.

The constriction of the incident light rays increases the depth of focus, featuring a pinhole effect. The engagement means may be provided by two handles (25) shaped with curved proximal ends joined to the peripheral edge of said diaphragm (21) and having substantially circular section with a diameter between 80µm and 800µm or two handles (25') of the same material as the diaphragm and constituting an extension of this edge, or even one elongated platform (30) whose center is located in the small-diameter circular opening (21").

## Description

### Field of Application

The present invention applies to the field of ophthalmology, and more specifically to the field of cataract surgery. It is an attempt to promote increased visual performance after implantation of an intraocular lens.

For a better understanding of this specification, we present below some technical terms used in the same:
Lens: - is an intraocular lens which is located between the iris and the vitreous humor. For full operation of the eye, a full transparency of said lens is required. This characteristic is achieved due to the absence of blood vessels and the compression of the plates that constitute it. The dimensions of the lens vary throughout life, with slow growth due to the constant formation of new lamellae.
Cataract: - is an eye disease that causes a loss of transparency of the lens, which can be triggered by several factors, including advanced age, ocular trauma, diabetes mellitus, uveitis, use of drugs, among other causes, causes a progressive reduction of visual acuity and may lead to blindness.
Accommodation: - Capacity of the human lens of changing its refractive power, with the purpose of setting the focus to different distances, based on a change of curvature of the lens. There is a progressive decline of said capacity with advancing age. Said mechanism is completely eliminated after cataract surgery.
Cornea: - transparent front part of an eye, which together with the sclera makes up the outer layer of the eye. For full operation it must be transparent and with a regular surface curvature. It is located in the anterior polar region of the eyeball. The cornea and lens make up the main refractive elements of the eye. Their purpose is to focus the incident light in the plane of the retina.
Pinhole effect: - Principle of Optics which is based on expanding the depth of field caused by the constriction of incident light rays in an optical system by a small diameter opening an opaque screen.
Stereopsis: - is the brain's ability to interpret two slightly different images coming from each eye and promote the fusion of these images, creating depth perception. To have stereopsis, it is necessary that the images interpreted by the brain are very similar to each other.
Iris: - intraocular thin circular structure, which act as a diaphragm. It controls the amount of light energy reaching the retina by increasing or reducing its central hole (pupil). The diaphragms of cameras have been developed in an attempt to simulate the operation of this structure.
Ciliary sulcus: - refer to an anatomical intraocular space between the posterior surface of the base of the iris and and the anterior surface of the ciliary body.
Uveal tissue: - vascular middle layer of the eye, being a tissue divided in three parts of eye iris, ciliary body and choroid. Its comprises widely vascularized muscle cells being present in its composition.

### State of the Art

The human eye has a lens inside called natural lens. It is a transparent structure that aims to refract the incident light rays and focus them on the retina. With advancing age there is a physiological loss of transparency of the lens, called a cataract. When this loss of transparency becomes significant a cataract surgery is indicated.

During cataract surgery the human lens is extracted and a new clear artificial lens is implanted into an eye, thereby restoring the ability to focus the rays on the retina. This artificial lens inside the eye remains throughout life. Because it is an artificial lens, it has no mechanism for adjusting the focus to different distances. Therefore, after a successful surgery, the patient will have a good distance vision, but will have to wear glasses to achieve near focus, or vice versa.

The attempt to achieve spectacle independence after cataract surgery has boosted the development of multifocal intraocular lenses. These models promote fair spectacle independence, but causes reduced contrast sensitivity and optical quality, in addition, optical phenomena such as halos and glare can cause loss of visual acuity.

Another commonly used strategy is monovision. The surgeon implants a monofocal lens in one eye with its power calculated for distance vision. The contralateral eye is implanted with a monofocal lens aiming near vision. With binocular vision the patient can perform most of their activities without the use of glasses, but this strategy causes a reduction of stereopsis (the ability to see in 3D) and binocular contrast sensitivity.

Another important method, but not associated with cataract surgery is the use of optical devices that operate based on the Pinhole effect. They are called "small aperture". They are implanted inside the cornea, and through the pinhole effect, an increased depth of focus is promoted. These devices are implanted before the development of cataracts, when a physiological loss of accommodation causes limitation of near vision (presbyopia).

This procedure has several limitations, such as lack of knowledge about long-term biocompatibility of the implant material, need to perform a surgery on a perfectly normal structure, among others. Furthermore it blocks the viewing of other intraocular structures and may hinder future ophthalmic surgery such as cataract surgery case, and may even hinder routine examination of the interior of the eye.

Thus, for example, patent US 4 955 904 titled "Masked intraocular lens and method for treating a patient with cataracts" describes an intraocular lens configured to provide an increased depth of field, allowing focus on both near and distant images without the need for glasses. To do so, it uses the so-called "pinhole effect", whereby the image formed on the retina is always clear regardless of distance in which the object is observed. The intraocular lens described in this patent is provided with an opaque mask in whose center a transparent region with a diameter between 1 and 3 mm is provided and the external diameter of this mask ranges between 4 and 6 mm for an optical part of 7 mm. Despite being based on the pinhole effect, this patent relates to an intraocular lens with refractive power. Another feature that differentiates it is that the area outside the opaque region be transparent.

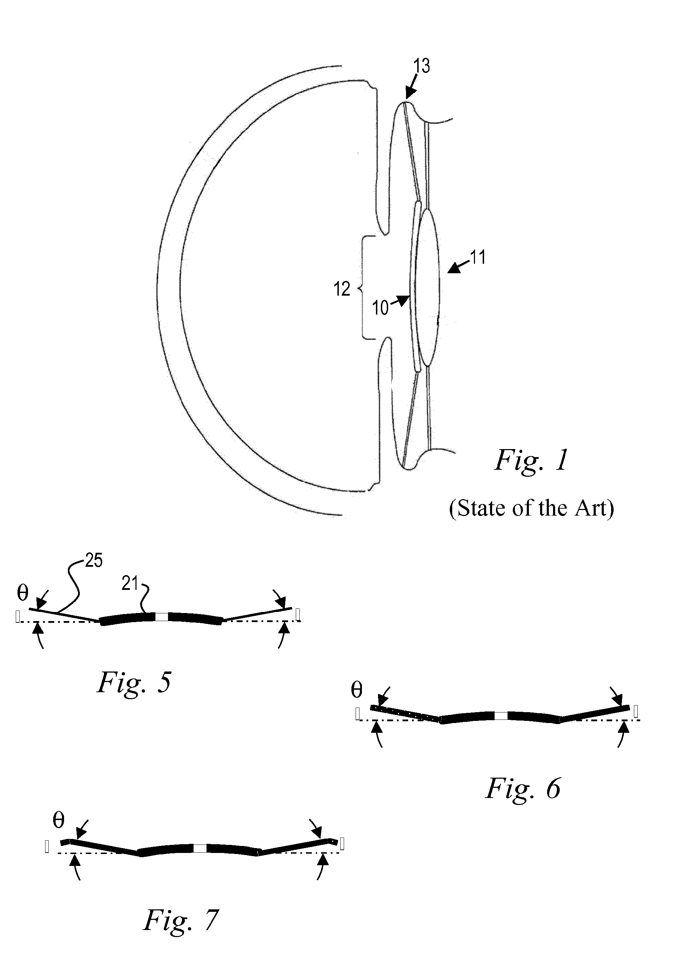

Patent application US 2012/0109294 entitled "Vision Correction System" teaches to overcome the shortcomings of residual lens already in place through the provision of a supplemental lens juxtaposed with the front surface of the lens previously deployed. Functional said vision correction system has an extra lens, with its anterior surface convex and posterior surface concave, as illustrated in Fig.1. As illustrated, said supplemental lens 10 is located behind the iris 12 and has a larger diameter than lens 11 previously deployed. The contact between both lenses 10, 11 is minimized by a design of the concave posterior surface of the supplemental lens 10. The supplemental lens 10, which is held in place by placing two handles 14 in the ciliary sulcus 13, is configured to have a refractive power that corrects said residual faults.

Like the previous reference, it is also a lens with refractive power. In contrast thereto, the lens of said application is transparent to visible light and is not based on a pinhole effect.

### Objectives of the Invention

In view of the above, the invention's main objective is to provide an intraocular implant to a person who has undergone cataract surgery with implantation of a standard intraocular lens, whereby the implant provides an increased depth of focus, allowing both near vision as far. Another objective of this invention is to minimize the impact of aberrations of low and high-order visual quality, thus promoting an improvement in visual acuity. Yet another objective is to eliminate the need for glasses by extending the depth of focus of a monofocal intraocular lens.

### Brief Description of the Invention

The invention relates to an intraocular implant comprising a diaphragm of opaque material with a small diameter central opening based on the pinhole effect, whereby the constriction of the light rays that reach an optical system promotes enlargement of the depth of focus.

According to another feature of the invention, the implant of the invention is housed inside the eye in a region preceding the pre-existent intraocular.

According to another aspect of the invention, the implant of the invention, blocks visible light and is totally transparent to light in the infrared spectrum. Thus, although blocking the view of the fundus through direct examination by light in the visible spectrum, the implant will allow an inspection of intraocular structures - such as anatomical details of the retina - by using devices based on infrared light.

According to another aspect of the invention, the implant comprises handles produced from a relatively flexible material, with thin circular cross-section which will prevent damage to the ciliary sulcus uveal tissue.

According to the invention a small aperture (pinhole) intraocular implant to increase depth of focus is proposed which comprises a diaphragm juxtaposed to a front surface of a lens implanted previously. Thediaphragm's anterior surface is convex and its posterior surface is concave. The diaphragm is maintained in position by inserting its engaging means on the ciliary sulcus.

It is suggested that said diaphragm is opaque to a visible light spectrum and transparent to light in an infrared range, means being provided with a passage for visible light in its central region. The visible light spectrum ranges from 390 nanometers to 700 nanometers. The wavelength range of infrared light is approx. from 750 nanometers to 1.000.000 (one million) nanometers. Therefore, the human eye is not sensitive to infrared light. The transparency to IR-light can be achieved by the use of a specific combination of dyes incorporated into the matrix of the synthetic material used in this implant. Since modern ophthalmic equipments used to examine the retina operate with infrared light, the examination can be normally performed with no interference by the suggested implant.

According to an embodiment of the invention, said light passage means can be provided by a hole located substantially in the center of the diaphragm. This central opening, with a small diameter, allows the passage of only the paraxial visible light rays through the diaphragm, extending depth of focus and neutralizing optical aberrations. This way, visual acuity is improved.

According to the foregoing embodiment the diameter of said through hole can be between 1 mm and 2.5 mm. The diameter of the hole, determines the percentage of light rays which will pass. Any diameter within this range is able to extend depth of focus and neutralize aberrations. Therefore the diameter of the central opening shall be determined on an individual basis, based on the characteristics of each patient, such as corneal aberrations and amount of depth of focus which should be induced.

According to any of both foregoing embodiments said through hole can be configured in the shape of a straight cylinder with a circular cross section or ellipsoidal cross section. Depending on the shape of the pupil, which is not always perfectly rounded, an ellipsoidal cross section shape of the hole may be more appropriate for specific patients.

According to the first foregoing embodiments said through hole can be configured in the shape of a straight truncated cone. This may induce less light diffraction, compared to the straight cylinder configuration.

According to a further embodiment edges and/or the outer line of said through hole can be rounded. This may facilitate folding the implant during implantation, compared to sharp edges.

According to a further embodiment said means for passage of light can be provided by a substantially circular transparent region located substantially at the center of the diaphragm. This small diameter central opening characterizes the pinhole implant, and is able to extend depth of focus by allowing only the paraxial rays to pass through the implant.

According to a further embodiment the diameter of the diaphragm can be in a range between 4mm and 7mm. If the diameter is smaller than 4mm, it may allow passage of light around it. This would decrease the effectiveness of the pinhole effect which is induced by the proposed implant. If the diameter is larger than 7mm, the process of folding the implant would become difficult, and a larger incision would be necessary to insert it inside the eye. According to a further embodiment said diaphragm thickness can be between 100µm and 900µm. If the thickness is less than 100µm, the implant would be too flexible, and would not hold its position inside the eye, with higher chances of dislocation and decentration. If the thickness is greater than 900µm, the process of folding the implant would become difficult, and a larger incision would be necessary to insert it inside the eye. Also there would be more contact of the implant with the intraocular tissues, which should be avoided.

According to a further embodiment said engaging means can comprise at least two handles (also called haptics) shaped with curved proximal ends joined to a peripheral edge of said diaphragm, said handles can have substantially circular cross sections with a diameter between 80µm and 800µm. The handles are the elements which ensure good centration and stabilization of the implant inside the eye. Therefore, if they are too thin, the implant could dislocate inside the eye, because of lack of mechanical strength. If they are too thick, a larger incision would be necessary to insert it inside the eye. Also there would be more contact of the implant with the intraocular tissues, which should be avoided.

According to the foregoing embodiment the material of said handles can be selected from the group comprising polymethylmethacrylat (PMMA), polyimide and prolene. Prolene is a synthetic, monofilament, non-absorbable polypropylene. It is indicated for skin closure and general soft tissue approximation and ligation. Its advantages include minimal tissue reactivity and durability. Disadvantages include fragility, high plasticity, high expense, and difficulty of use compared to standard nylon sutures. PMMA is an acrylic polymer, which offers greater mechanical strength. These materials are widely used as handles for the currently available intraocular lenses. All of these materials are very biocompatible.

According to a further embodiment the material of said diaphragm can be selected from a group comprising acrylic hydrophilic, hydrophobic acrylic, silicone and polymethylmethacrylat (PMMA). These materials are widely used in the optic portion of the currently available intraocular lenses. All of these materials are very biocompatible.

According to a further embodiment said engagement means can comprise at least two handles of curved shape, with proximal ends constituting extensions of a perimetric edge of said aperture, said handles can be made of the same material as the diaphragm. This is a common design of intraocular lenses, in which all parts of the lens are made of the same material. Therefore, during the manufacturing process the artificial lens is sculpted from a single block of material, instead of having the handles (haptics) inserted into the optic part of the lens. Currently, this is the most common type of intraocular lens design.

According to a further embodiment said engaging means can comprise an elongated platform in which said diaphragm is located at a substantially central position. This design is also applied in modern intraocular lenses. Instead of having elongated arms to hold the lens in place, this design is based on a platform-shape implant, which is kept in place by the contact of the edge of this platform with the ocular tissues.

According to any of both foregoing embodiments said material is selected from a group comprising hydrophilic acrylic, hydrophobic acrylic, silicone and polymethylmethacrylat (PMMA). These are the most biocompatible materials currently used in modern intraocular lenses. All of them, except the PMMA, are foldable, which allows insertion in the eye through a smaller incision.

### Brief Description of the Figures

The invention will be better understood from the detailed description which follows of a non-limiting exemplary embodiment and the figures related to it, wherein:
- **Figure 1** illustrates a supplemental intraocular lens known deployed in front of a preexisting monofocal lens;
- **Figure 2** illustrates a front view of a first embodiment of the intraocular implant configured in accordance with the principles of the present invention;
- **Figure 3** illustrates a front view of a second embodiment of the intraocular implant configured in accordance with the principles of the present invention;
- **Figure 4** illustrates a front view of a third embodiment of the intraocular implant configured in accordance with the principles of the present invention;
- **Figure 5** is a sectional view of the intraocular implant shown in front view in figure 2;
- **Figure 6** is a sectional view of the intraocular implant shown in front view in figure 3;
- **Figure 7** is a sectional view of the intraocular implant shown in front view in figure 4;
- **Figure 8** illustrates a front view of another embodiment according to the invention;
- **Figure 9** illustrates a front view of another embodiment according to the invention;
- **Figure 10** illustrates a front view of another embodiment according to the invention.

### Detailed Description of the Invention

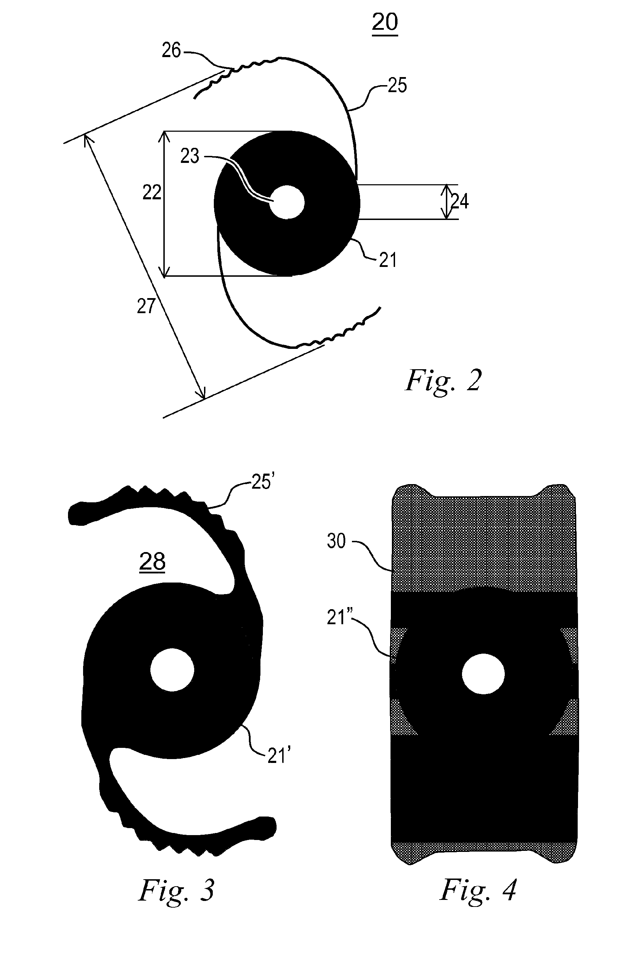

An embodiment of an implant according to the invention is described below in detail through exemplary achievements. Details are illustrated in Figures 2 and 5, which represent a first embodiment of the invention.

Anintraocular implant comprises a body 20 shaped like a concave-convex diaphragm 21 ,made of rigid or flexible material, preferably flexible, the material or a coating or coloring layer is opaque to visible light, preferably black colored material or a black coating22 with a diameter of between 4 mm and 7 mm, preferably between 5.5 mm and 6.5 mm.

In its central region a passage means 23 for visible light rays with a diameter 24 of between 1 mm and 2.5 mm, preferably between 1.4 mm and 2.0 mm is arranged, which allows a passage of light rays which, due to the pinhole effect, are sharply focused on the retina, inversely proportional to the diameter of the passage. The thickness of said diaphragm is between 100µm and 900µm, preferably between 200µm and 400µm.

Said pinhole effect is commonly used by photographers. Several low-cost cameras have monofocal lenses. Thus it is possible to produce photos with clarity at different distances when the diaphragm of the lens is reduced in diameter.

Also according to the figure, the implant of the invention has handles 25 produced by a material with sufficient strength for fixing the implant, being thin and having a substantially circular cross- section with a diameter between 80µm and 800µm, preferably 200µm, thus to prevent damage of the tissue of the uveal ciliary sulcus.

As illustrated, said loops or handles have a curved shape with their proximal ends attached to the peripheral edge of said diaphragm 21, the distance 27 between their distal ends 26 can be between 10mm and 15mm. The distal ends 26 can have an undulated or zigzag shape.

According to the sectional view of Figure 5, said handles/loops form an angle θ with respect to the main plane of the diaphragm, whose value is between 4° and 12°, preferably about 10°, said angle θ having a function of keeping the Iris diaphragm away to avoid body contact with the iris tissue.

The loops' / handles' material must not be too rigid or too flexible. In the first case, the excessive rigidity hinders the implant into the eye, and if it is too flexible or elastic, the implant does not remain fixed in position. The main materials used can be PMMA (polymethylmethacrylate), polyimide and/or prolene.

Said light passage means 23 may be provided by an opaque material through opening in the diaphragm, according exemplified in Fig.5, or by modifying the characteristics of this material make it transparent in a substantially circular region.

In the case of said means being constituted by an opening, it can be configured with the shape of a straight cylinder of circular cross section or ellipsoidal cross section, or even in the form of a straight truncated cone. In an alternative embodiment, the edges of the opening can be rounded to avoid diffraction effects.

Also in accordance with the principles of the invention, said aperture is opaque with respect to visible light and ultraviolet light, but is transparent to light in the infrared range. This allows viewing the internal structures of the eye such as the retina layers through usage of am equipment operating in this spectral range.

As the optic material is preferably flexible, the implant can be implanted in a manner similar to an intraocular lens through a small corneal incision.

Other settings that can be adopted for the implant of the invention are described below.

A one-piece model is illustrated in front view in Figure 3 and in section in Figure 6. The diaphragm's body 21 and the handles 25' are made of the same material. The implant comprises curved handles 25' whose proximal ends are extensions of said aperture body's perimeter edge, said loops 25' being made of the same lens material. As in the embodiment described above, the handles 25'are developed in an angle θ of about 10 degrees with respect to the body 21 of the diaphragm to minimize contact with the iris's fabric.

A Platform model is illustrated in front view in Figure 4 and in section in Figure 7, where the implant has elongated shape, with a central body aperture 21' which is in continuation with an anchoring structure 30 being represented by a platform or sheet material.

Given an implant formed according to the first embodiment, illustrated in Figures 2 and 5, suitable materials for the body of the diaphragm 21 would be hydrophilic acrylic, hydrophobic acrylic, silicone and/or PMMA, preferably acrylic hydrophilic. The most suitable materials for the handles 25 would be PMMA (polymethylmethacrylate), polyimide and prolene, preferably PMMA.

Considering an implant configured according to the second embodiment illustrated in Figures 3 and 6, the most suitable materials for the whole-body handles 25' would hydrophilic acrylic, hydrophobic acrylic, silicone and/or PMMA, preferably hydrophilic acrylic.

Further, an implant configured according to the third embodiment illustrated in Figures 4 and 7, suitable materials for the platform 30 would be silicone, acrylic hydrophobic and hydrophilic acrylic, preferably silicone.

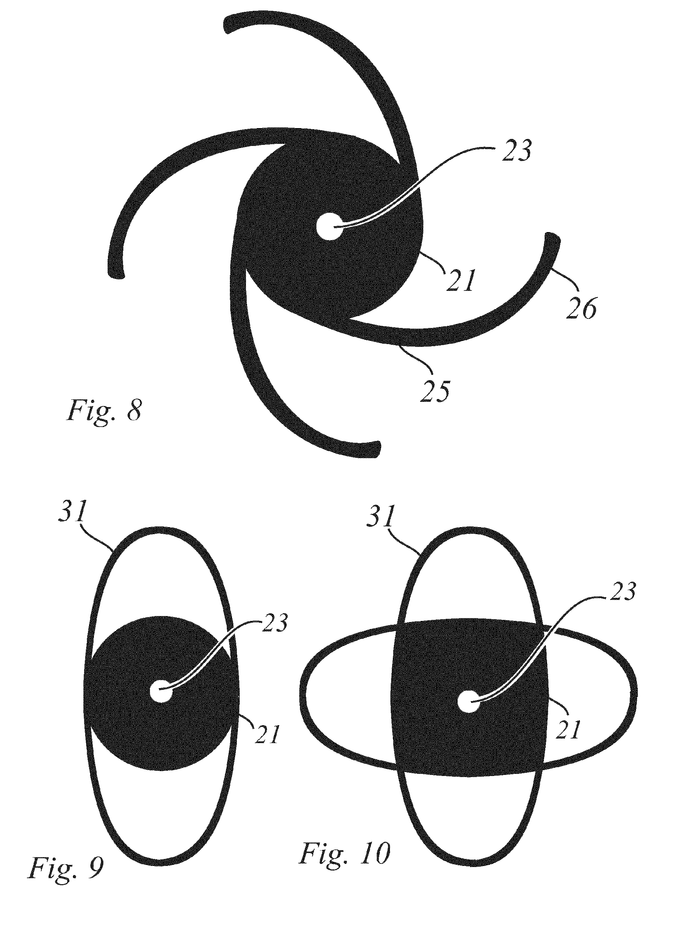

In Figure 8 an embodiment of an implant with a circular diaphragm 21 with hole 23 and four handles 25 is depicted. The implant of Fig. 8 is a modification of the embodiment of Fig. 3. The handles 25 can also comprise an undulated or zigzag distal end part 26 for improving fastening characteristics and flexibility. Another embodiment can also comprise three or five handles 23.

In Figures 9 and 10 further embodiments of an implant according to the invention are displayed. The implant comprises a diaphragm 21 being opaque to visible light and transparent to IR-light with a circular hole 23 in its center and two, three, four or even more loops 31. The loops 31 are formed in a bended curvature and both ends of each loop 31 is attached to diaphragm 21. Thus a lightweight and securely fastening implant is provided, which can be considered as a combination of first and second embodiment.

In summary, due to the fact that the implant does not provide a lens refractive power, but work as a small diaphragm opening, the implant of the present invention has significant advantages compared with the known art.

Regarding the use of multifocal lenses, such advantages are:
- Does not cause significant reduction in contrast sensitivity;
- Does not cause adverse phenomena such as halos and glare;
- Does not require special conditions for their full operation as stable tear film, ideal centering of the intraocular lens, etc.;
- Manufacture process is much simpler and cheaper, and
- Easily reversible by explantation of the lens through the same incision by which it was located.

The main advantages of the invention compared to monovision are as follows:
- Does not cause reduction of stereopsis;
- Does not cause significant reduction in contrast sensitivity bilaterally;

Finally, the implant of the invention has the following advantages over the corneal inlays:
- No need for additional surgery in a normal structure;
- Its biocompatibility is proven; - There is no cosmetic change;
- Its centering in relation to the visual axis is very easy
- It is possible to carry out imaging of the fundus with infrared light, and
- It is easily reversible by explantation of the implant through the same incision through which it was deployed.

According to said invention the diaphragm is opaque to a visible light spectrum and transparent to light in the infrared range and is equipped with passage means 23 of visible light in its central region, such as a through hole whose diameter is between 1 mm and 2.5 mm.

The constriction of the incident light rays increases the depth of focus, featuring a pinhole effect. The engagement means may be provided by two handles 25 shaped with curved proximal ends joined to the peripheral edge of said diaphragm 21 and having substantially circular section with a diameter between 80µm and 800µm, or two handles 25' of the same material as the diaphragm and constituting an extension of this edge, or even one elongated platform 30 whose center is located in the small-diameter circular opening 21".

Although the present invention has been described in connection with preferred forms of embodiment, it should be understood that it is not intended to limit the invention to those particular rules. On the contrary, it is intended to cover all alternatives, modifications and equivalents as possible within the spirit and scope of the invention which is defined by the set of claims that follows.

## Claims

1. **Small aperture (pinhole) intraocular implant to increase depth of focus** comprising a diaphragm (21, 21', 21 ") juxtaposed to a front surface of a lens (11) implanted previously, having its anterior surface convex and posterior surface concave maintained in position by inserting an engaging means (25, 25', 25") on the ciliary sulcus (13), **characterized in that** said diaphragm (21, 21', 21") is opaque to a visible light spectrum and transparent to light in an infrared range, means being provided with a passage for visible light in its central region.

2. Implant according to claim 1, **characterized in that** said light passage means is provided by a through hole (23) located substantially in the center of the diaphragm (21, 21', 21").

3. Implant according to claim 2, **characterized in that** the diameter of said through hole (23) is between 1 mm and 2.5 mm.

4. Implant according to claim 2 or 3, **characterized in that** said through hole (23) is configured in the shape of a straight cylinder with a circular cross section or ellipsoidal cross section.

5. Implant according to claim 2 or 3, **characterized in that** said through hole (23) is configured in the shape of a straight truncated cone.

6. Implant according to any of claims 2 to 5, **characterized in that** the edges of said through hole (23) are rounded.

7. Implant according to any of the aforementioned claims, **characterized in that** said means for passage of light are provided by a substantially circular transparent region located substantially at the center of the diaphragm (21, 21', 21 ").

8. Implant according to any of the aforementioned claims, **characterized in that** the diameter (22) of the diaphragm (21, 21', 21") is between 4mm and 7mm.

9. Implant according to any of the aforementioned claims, **characterized in that** said diaphragm thickness is between 100µm and 900µm.

10. Implant according to any of the aforementioned claims, **characterized in that** said engaging means comprise at least two handles (25) shaped with curved proximal ends joined to the peripheral edge of said diaphragm (21), said handles (25) having substantially circular cross sections with a diameter between 80µm and 800µm.

11. Implant according to claim 10, **characterized in that** the material of said handles (25) is selected from a group comprising polymethylmethacrylat (PMMA), polyimide and prolene.

12. Implant according to any of the aforementioned claims, **characterized in that** the material of said diaphragm (21) is selected from a group comprising acrylic hydrophilic, hydrophobic acrylic, silicone and polymethylmethacrylat (PMMA).

13. Implant according to any of the aforementioned claims, **characterized in that** said engagement means comprise at least two handles (25') of curved shape, with proximal ends constituting extensions of a perimetric edge of said aperture (21'), said handles (25') being made of the same material as the diaphragm.

14. Implant according to any of the aforementioned claims, **characterized in that** said engaging means comprise an elongated platform (30) in which said diaphragm (21") is located at a substantially central position.

15. Implant according to claim 13 or 14, **characterized in that** said material is selected from a group comprising hydrophilic acrylic, hydrophobic acrylic, silicone and polymethylmethacrylat (PMMA).
